# EUROPÄISCHE PATENTSCHRIFT

(11) **EP 2 816 987 B1**
(45) Veröffentlichungstag und Bekanntmachung des Hinweises auf die Patenterteilung: **08.06.2016**
(21) Anmeldenummer: 12711560.8
(22) Anmeldetag: 09.03.2012
(51) Int. Cl.: A61F 13/14

(54) **STILLEINLAGE**
BREAST FEED PAD
COUSSINET D'ALLAITEMENT

(30) Priorität: 24.02.2012 DE 202012001829 U
(43) Veröffentlichungstag der Anmeldung: 31.12.2014
(73) Patentinhaber: MAPA GmbH, 27404 Zeven (DE)
(72) Erfinder: LADIGES, Elke, 27412 Hepstedt (DE)
(74) Vertreter: Hauck Patentanwaltspartnerschaft mbB
(86) Internationale Anmeldenummer: PCT/EP2012/001060
(87) Internationale Veröffentlichungsnummer: WO 2013/123952

(56) Entgegenhaltungen:
- WO-A1-96/41601
- WO-A1-2004/054495
- DE-A1- 19 646 789
- US-A- 6 039 629

## Beschreibung

Die Erfindung bezieht sich auf eine Stilleinlage zum Aufsetzen auf die Brust einer Frau.

Stilleinlagen dienen dazu, den Abfluss von Muttermilch von den Brüsten stillender Frauen zu verhindern.

Abfließende Muttermilch verunreinigt die Kleidung und wird als unangenehm empfunden. Bekannt sind absorbierende Stilleinlagen mit einer flüssigkeitsundurchlässigen Außenschicht, die auf die Brust aufgesetzt werden und abströmende Muttermilch auffangen. Diese haben den Nachteil, dass sie bauschig sind und stark auftragen. Auch wird das Anliegen des feuchten absorbierenden Kissens auf der Haut als unangenehm empfunden.

Die WO 2009/043007 A1 betrifft eine wieder verwendbare, waschbare, klebende Abdeckung für Verwendung durch laktierende Frauen in Kombination mit einer absorbierenden Stilleinlage. Die Abdeckung weist eine Vielzahl von laminierten Schichten aus Silikonzusammensetzungen auf. Eine äußere Schicht hat eine wesentliche größere Härte als eine innere Schicht. Die äußere Schicht verleiht der Abdeckung Steifigkeit und die Silikonzusammensetzung mit der geringeren Härte an der Innenseite klebrige Eigenschaften, so dass die Abdeckung eine Fixierung ohne zusätzliche Befestigung an der Kleidung ermöglicht.

Bekannt ist, dass ein leichter Druck auf die Brustwarze das Abströmen von Muttermilch steuert und begrenzt. Aus der WO 03/105616 A1 ist eine Vorrichtung zum Steuern der Milchabsonderung bekannt. Diese Vorrichtung weist ein dünnes, flexibles und atmungsfähiges, flüssigkeitsundurchlässiges und mehrschichtiges Schild auf, das sich leicht an die Form der menschlichen Brust anpasst. Die Vorrichtung ist gemeinsam mit einem Büstenhalter benutzbar, um die Formung des Schildes zu unterstützen und einen zusätzlichen Druck auf die Brustwarze der Trägerin auszuüben, um spontane Milchabsonderungen zu verhindern.

Das Schild ist eine dünne Membran mit einer inneren Oberfläche und einer äußeren Oberfläche. Es ist aus einem Material hergestellt, dass hinreichend dehnbar in beliebige Richtungen ist, so dass durch einen geringen Druck an die Form der Brust angepasst wird. Das Schild hat eine klebrige innere Oberfläche, die es ermöglicht, die Lage beizubehalten, ohne Klebemittel einzusetzen oder an der äußeren Kleidung sowie einem Büstenhalter befestigt zu werden. Das Schild ist vorgeformt, so dass es im Querschnitt eine elliptische Oberfläche hat, die es ermöglicht, das Schild enger an die Form der Brust anzupassen. Das Material des Schildes ist hinreichend durchscheinend, so dass es beinahe unsichtbar ist, wenn es unter der Kleidung angeordnet ist. Die Dicke des Schildes ist so gewählt, dass es das Brustprofil der Trägerin nahezu nicht verändert. Das Schild hat eine äußere Schale aus Silikongummi und eine innere Membran aus einer Silikongummizusammensetzung, die auf die innere Oberfläche der äußeren Schale auflaminiert ist. Die äußere Schale und die innere Membran haben identische elliptische Krümmungen, wobei die äußere Schale eine konvexe Oberfläche und die innere Membran eine konkave Oberfläche aufweisen.

Die US 6,200,195 B1 beschreibt eine wiederholt verwendbare, waschbare, anhaftende Einlage zum Haften am menschlichen Körper, die einen Einlagenkörper aus einem Silikongummi aufweist und eine Klebeschicht aus einem Silikongel, die aus einem Stück mit dem Einlagenkörper gebildet ist, um eine anhaftende Oberfläche zur Verfügung zu stellen. Der Einlagenkörper und die anhaftende Schicht aus Silikongel sind vernetzt und nahtlos miteinander verbunden, wobei die anhaftende Schicht aus Silikongel ihr Haftvermögen je nach Gebrauch für ca. zwei Monate beibehält. Diese Einlage ist wiederholt direkt an der Haut der Trägerin anklebbar. Sie ist kuppelförmig.

Bei den bekannten Einlagen zum direkten Ankleben an die Haut, die nicht mit einem Absorptionskissen versehen sind, kann es dennoch zum Abströmen von Milch kommen.

Davon ausgehend liegt der Erfindung die Aufgabe zugrunde, eine Stilleinlage zur Verfügung zu stellen, die das ungewollte Abströmen von Milch besser verhindert.

Die Aufgabe wird durch eine Stilleinlage mit den Merkmalen von Anspruch 1 gelöst. Vorteilhafte Ausgestaltungen der Stilleinlage sind in Unteransprüchen angegeben.

Die erfindungsgemäße Stilleinlage zum Aufsetzen auf die Brust einer Frau hat einen zwecks Anpassung an die Form einer Brust dünnwandigen, flexiblen Einlagenkörper mit einem umlaufenden Außenrand, einer inneren Oberfläche zum Anlegen an die Brust und einer äußeren Oberfläche, wobei von der inneren Oberfläche des Einlagenkörpers eine Erhebung zum Zwecke des Eindrückens einer Brustwarze vorsteht.

Der erfindungsgemäßen Stilleinlage liegt die Erkenntnis zugrunde, dass sich herkömmliche Stilleinlagen nicht ideal an eine Brust anschmiegen, so dass sie die Brustwarzen oftmals gar nicht oder nicht hinreichend eindrücken, um ein Abfließen von Muttermilch in dem gewünschten Ausmaß zu verhindern. Für ein hinreichendes Eindrücken der Brustwarze ist der Einlagenkörper der erfindungsgemäßen Stilleinlage im Zentrum mit einer von der inneren Oberfläche vorstehenden Erhebung versehen. Wenn die Stilleinlage an der Brust angebracht ist, drückt die Erhebung die Brustwarze ein, auch wenn die Stilleinlage nicht ideal an der Brust anliegt. Da die Stilleinlage an der Brust angebracht ist und die Brustwarze eindrückt, bewirkt sie einen natürlichen Verschluss der Milchdrüsen. Dieser kann durch Abnehmen der Stilleinlage geöffnet werden. Die Erhebung erleichtert das Positionieren der Stilleinlage auf der Brust bzw. der Brustwarze.

Gemäß einer bevorzugten Ausgestaltung ist die Erhebung eine Dickstelle des Einlagenkörpers. Die Ausgestaltung der Erhebung als Dickstelle hat den Vorteil, dass die Stilleinlage an der Erhebung eine verringerte Flexibilität aufweist. In Folge dessen kann die Erhebung beim Eindrücken der Brustwarze nicht ausweichen. Dies trägt dazu bei, dass die Brustwarze hinreichend eingedrückt wird, um einen Milchabfluss zu unterbinden. Bei dieser Ausgestaltung ist bevorzugt der Einlagenkörper außerhalb der Erhebung besonders dünnwandig bzw. flexibel ausgestaltet, so dass er sich besonders gut an die Brust anschmiegt. Auch dies trägt dazu bei, dass die Erhebung korrekt platziert ist und die Brustwarze hinreichend eindrückt. Zudem wird durch die Anpassung an die Brust erreicht, dass das Tragen der Stilleinlage unter der Kleidung nicht auffällt.

Grundsätzlich ist es aber auch möglich, die Erhebung als nach innen gewölbten zentralen Bereich eines dünnwandigen Einlagenkörpers mit im wesentlichen konstanter Wandstärke im Bereich der Erhebung und in den anderen Bereichen auszugestalten. Die solchermaßen gebildete Erhebung kann aufgrund ihrer kompakten Form hinreichend stabil ausgeführt werden, um die Brustwarze einzudrücken.

Gemäß einer Ausgestaltung ist der Einlagenkörper kuppelförmig gewölbt. Die kuppelförmige Wölbung ist beispielsweise gegeben, wenn der Einlagenkörper die Form einer Halbkugelschale oder eines kleineren Teils einer Kugelschale oder eine Glockenform aufweist. Die kuppelförmige Wölbung des Einlagenkörpers erleichtert die Anpassung an die Form der Brust.

Gemäß einer weiteren Ausgestaltung hat der Einlagenkörper um die Erhebung herum eine konkave innere Oberfläche und/oder hat der Einlagenkörper eine konvexe äußere Oberfläche.

Gemäß einer weiteren Ausgestaltung nimmt die Wandstärke des Einlagenkörpers vom umlaufenden Außenrand bis zum äußeren Rand der zentralen Erhebung allmählich zu. Dieser Wandstärkenverlauf bewirkt zugleich eine vorteilhafte Anpassung an die Form der Brust und Abstützung der zentralen Erhebung, so dass diese die Brustwarze hinreichend eindrücken kann, ohne nachzugeben.

Gemäß einer Ausgestaltung ist die Erhebung zumindest teilweise konvex gekrümmt. Die konvexe Krümmung begünstigt ein angenehmes Tragegefühl. Bevorzugt ist die Erhebung insgesamt konvex gekrümmt. Vorzugsweise ist die Erhebung nur schwach konvex gekrümmt, damit die Brustwarze eine annähernd flache Auflagefläche hat und nicht seitlich abrutscht. Gemäß einer bevorzugten Ausgestaltung hat die Erhebung die Form eines Kugelsegments. Gemäß einer weiteren Ausgestaltung beträgt der Krümmungsradius der konvexen Erhebung mindestens 5mm. Weiterhin bevorzugt beträgt er maximal 15mm.

Gemäß einer weiteren Ausgestaltung ist die Erhebung nur teilweise konvex gekrümmt und im Scheitelbereich abgeflacht, um dort eine breite Anlage für die Brustwarze zu bieten. Der Übergang zwischen der Abflachung und dem konvex gekrümmten Mantel ist bevorzugt gerundet.

Grundsätzlich ist es auch möglich, die Erhebung kegelstumpfförmig oder zylindrisch auszubilden, wobei der Übergang vom konischen bzw. zylindrischen Mantel zum abgeflachten Ende vorzugsweise gerundet ist.

Gemäß einer weiteren Ausgestaltung ist der Übergang von der Erhebung zu dem diese umgebenden Bereiche der inneren Oberfläche gerundet, um ein angenehmes Tragegefühl zu fördern. Der Krümmungsradius des gerundeten Übergangs von der Erhebung zu dem diese umgebenden Bereich ist bevorzugt nur schwach gekrümmt. Sie kann insbesondere einen Krümmungsradius von mindestens 5mm aufweisen. Bevorzugt beträgt dieser Krümmungsradius maximal 15mm.

Gemäß einer weiteren Ausgestaltung steht die Erhebung bezüglich der daran angrenzenden, inneren Oberfläche um 0,5 bis 5,0mm vor. In diesem Bereich der Abmessungen wird die Brustwarze in einem Ausmaß eingedrückt, der den Milchabfluss unterbindet, jedoch nicht als unangenehm empfunden wird. Weiterhin bevorzugt steht sie um 1,0 bis 2,0mm vor. Vorzugsweise steht sie etwa 1,5 bis 1,75mm vor.

Gemäß einer bevorzugten Ausgestaltung ist der Einlagenkörper dünnwandig bzw. hat eine so geringe Wandstärke, dass er anschmiegsam ist. Gemäß einer weiteren Ausgestaltung weist der Einlagenkörper im Bereich der Erhebung eine Wandstärke von 1,5 bis 10mm und/oder außerhalb der Erhebung eine Wandstärke von 0,3 bis 5mm auf. Hierdurch wird eine hinreichende Stabilität im Zentrum und eine geeignete Flexibilität außerhalb des Zentrums erreicht. Weiterhin bevorzugt weist der Einlagenkörper im Bereich der Erhebung eine Wandstärke 2,5 bis 7,5mm und/oder außerhalb der Erhebung eine Wandstärke von 0,5 bis 3mm auf.

Gemäß einer weiteren Ausgestaltung erstreckt sich die Erhebung vom Zentrum des Einlagekörpers in radialer Richtung 3 bis 12mm weit. Weiterhin bevorzugt erstreckt sie sich 4 bis 8mm weit.

Gemäß weiteren Ausgestaltungen ist der umlaufende Außenrand des Einlagenkörpers gekrümmt. Gemäß einer weiteren Ausgestaltung ist der Außenrand kreisrund oder elliptisch oder blütenförmig. Die blütenförmige Ausgestaltung hat eine Serie aus bogenförmigen Abschnitten des Außenrandes. Die Blütenform passt sich besonders gut an die Brust an. Sie verteilt den Anlagedruck auf eine größere Kontaktlinie, wodurch Abdrücke auf der Haut reduziert werden.

Gemäß einer anderen Ausgestaltung ist der Außenrand des Einlagenkörpers fingerförmig. Bei dieser Ausgestaltung erstrecken sich ausgehend von der zentralen Erhebung oder von einem die zentrale Erhebung umgebenden Randbereich geringförmige Streifen radial nach außen, wobei zwischen den Streifen Freiräume sind.

Gemäß einer weiteren Ausgestaltung steht die Erhebung im Zentrum von der inneren Oberfläche des Einlagenkörpers vor. Bei dieser Ausgestaltung befindet sich die Erhebung im Mittelpunkt des Einlagenkörpers, wenn dieser kreisrund ist. Wenn der Einlagenkörper elliptisch ist, befindet sich die Erhebung im Schnittpunkt der Haupt- und Nebenachse der Ellipse. Bei einem Einlagenkörper mit blütenförmigen oder fingerförmigem Außenrand oder einem Außenrand mit einer anderen, insbesondere unregelmäßigen Form befindet sich die Erhebung vorzugsweise im Schwerpunkt des Einlagenkörpers.

Gemäß einer bevorzugten Ausgestaltung weist die innere Oberfläche ein Haftmittel auf. Das Haftmittel ermöglicht ein einfaches Ankleben direkt an die Hautoberfläche. Hierdurch wird die Stilleinlage unverrückbar in der Position gehalten, in der die Erhebung gegen die Brustwarze drückt. Außerdem werden aufwendige Befestigungsmaßnahmen beispielsweise durch Einsatz zusätzlicher Klebemittel oder Befestigen der Stilleinlage an Büstenhalter vermieden. Die Anwendung wird hierdurch vereinfacht.

Gemäß einer weiteren Ausgestaltung weist die innere Oberfläche eine Haftschicht aus einem Haftmittel auf. Bevorzugt ist die innere Oberfläche ganzflächig mit der Haftschicht aus einem Haftmittel beschichtet.

Gemäß einer weiteren Ausgestaltung ist die Stilleinlage anschmiegsam und/oder hautähnlich und/oder nimmt keine Flüssigkeit auf. Bevorzugt nimmt sie keine Milch und/oder kein Wasser auf.

Dies ist vorteilhaft für den Tragekomfort der Stilleinlage. Die Eigenschaften können der Stilleinlage durch eine geeignete Gestaltung und/oder durch den Einsatz geeigneter Materialien verliehen werden. Sie können der Stilleinlage insbesondere durch eine entsprechende Ausgestaltung des Einlagenkörpers oder des Haftmittels oder des Einlagenkörpers und des Haftmittels verliehen werden.

Gemäß einer weiteren Ausgestaltung besteht der Einlagenkörper aus Silikongummi und/oder das Haftmittel aus einem Silikongel. Die genannten Materialien weisen eine geeignete Flexibilität und Anschmiegsamkeit auf und können hinreichend hautähnlich und keine Flüssigkeit aufnehmend ausgebildet sein. Grundsätzlich ist es möglich, die Stilleinlage mehrfach zu verwenden, wobei sie gegebenenfalls durch die mehreren Verwendungen gewaschen werden kann. Vorzugsweise ist die Stilleinlage lichtdurchlässig bzw. durchscheinend.

Der Einlagenkörper hat vorzugsweise einen Durchmesser im Bereich von 50mm bis 150mm. Vorzugsweise liegt der Durchmesser im Bereich von 70mm bis 100mm.

Die Haftschicht hat vorzugsweise eine Schichtstärke von 0,05 bis 1,0mm. Vorzugsweise beträgt die Schichtstärke 0,1 bis 0,3mm.

Die Erfindung wird nachfolgend anhand der anliegenden Zeichnungen eines Ausführungsbeispiels in erfindungsgemäßen Stilleinlagen näher erläutert. In der Zeichnung zeigen:
Fig. 1 die Stilleinlage in Seitenansicht;
Fig. 2 die Stilleinlage in der Draufsicht;
Fig. 3 die Stilleinlage in einem Schnitt senkrecht durch den Scheitel des Einlagenkörpers.

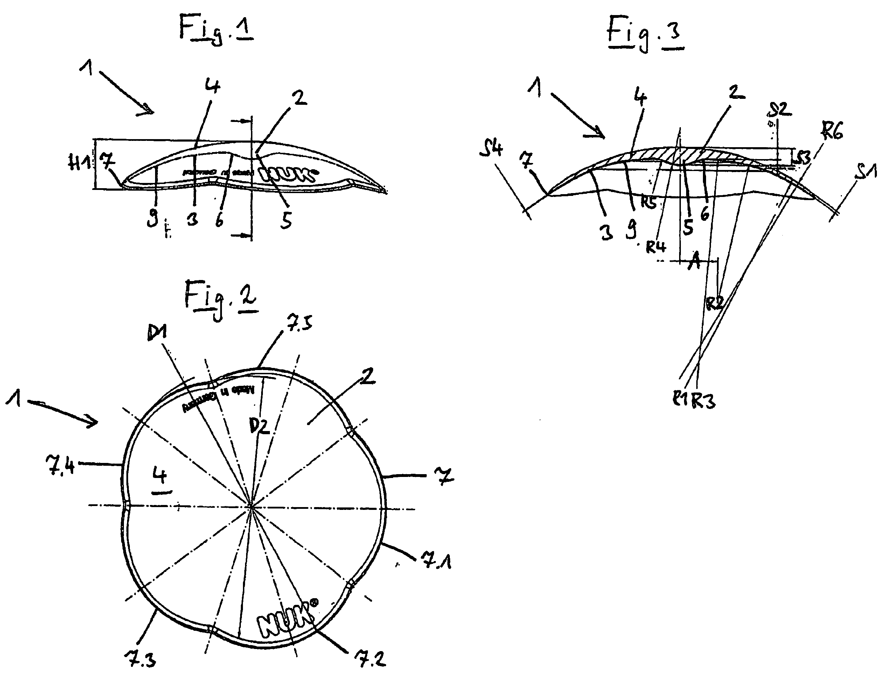

Gemäß Fig. 1 bis 3 hat die Stilleinlage 1 einen kuppelförmig gewölbten Einlagenkörper 2, der eine konkave innere Oberfläche 3 und eine konvexe äußere Oberfläche 5 aufweist. Im Zentrum endet die konkave innere Oberfläche 3 in einer konvexen Erhebung 5. Der Übergang von der Erhebung 5 zur inneren Oberfläche 3 weist eine Rundung 6 auf.

Der Einlagenkörper 2 hat einen gekrümmten, umlaufenden Außenrand 7. Der Außenrand 7 hat eine Serie bogenförmig gekrümmte Abschnitte 7.1 bis 7.5. Die Übergänge zwischen den bogenförmigen Abschnitten 7.1 bis 7.5 sind gerundet.

Die Wandstärke des Einlagenkörpers 2 nimmt vom Außenrand 7 zur Erhebung 5 hin allmählich zu. Am Außenrand 7 hat der Einlagenkörper 2 eine Wandstärke S1 von 0,65mm und am Rand der Erhebung 5 eine Wandstärke S2 von etwa 2mm. Am höchsten Punkt der Erhebung 6 hat er eine Wandstärke S3 von 5mm.

Der Einlagenkörper 2 hat folgende Radien:
R1=70mm, R2=40mm, R3=120mm, R4=10,7mm, R5=10mm, R6=66mm.

Vorzugsweise sind die Zentren der Radien R2 auf einem Kreis angeordnet, der um die in Fig. 3 vertikale Mittelachse in einem Abstand A von etwa 11mm umläuft.

Der Durchmesser D1 eines die Stilleinlage 1 umschreibenden Kreises beträgt etwa 80mm. Der Durchmesser D2 des in den umlaufenden Außenrand 7 einbeschriebenen Kreises beträgt etwa 75mm.

Die Höhe H1 der Stilleinlage von Scheitel der äußeren Oberfläche 4 bis zu den tiefsten Punkten des Außenrandes 7 beträgt etwa 15mm.

Auf die innere Oberfläche 3 des Einlagenkörpers 2 ist eine Haftschicht 9 aus Silikongel angebracht. Die Haftschicht 9 aus Silikongel hat eine Schichtstärke S 4 von 0,2mm bis 0,5mm.

Der Einlagenkörper 2 aus Silikon kann im Spritzgußverfahren oder im Compression Moulding-Verfahren oder Pressverfahren hergestellt werden.

Die Herstellung der Stilleinlage 1 mit einem Einlagenkörper 2 aus Silikon und einer Haftschicht 9 aus Silikongel an der inneren Oberfläche 3 kann auch so erfolgen, wie in der WO 03/105616 A1 oder der US 6,200,195 beschrieben. Die diesbezügliche Beschreibung in den beiden Patentdokumenten wird durch Bezugnahme in die vorliegende Anmeldung einbezogen.

Die Stilleinlage 1 wird durch auf die Brust aufgesetzt, wobei die Erhebung 5 gegen die Brustwarze gedrückt und die die Erhebung 5 umgebenden Bereiche an die Brust angeformt werden. Die Haftschicht 9 hält die solchermaßen positionierte Stilleinlage 1 auf der Haut fest. Der Milchabfluß wird effektiv verhindert. Die Stilleinlage 1 kann leicht abgezogen, gewaschen und erneuert werden.

Bei weiteren Ausführungsbeispielen weichen eine oder mehrere der Abmessungen S1, S2, S3, R1, R2, R3, R4, R5, R6, D1, D2, H1 maximal um ±50% ab. Vorzugsweise weichen sie maximal um ±20% ab.

## Patentansprüche

1. Stilleinlage zum Aufsetzen auf die Brust einer Frau mit einem zwecks Anpassung an die Form einer Brust dünnwandigen, flexiblen Einlagenkörper (2) mit umlaufendem Außenrand (7), einer inneren Oberfläche (3) zum Anlegen an die Brust und einer äußeren Oberfläche (4), wobei von der inneren Oberfläche (3) des Einlagenkörpers (2) eine Erhebung (5) zum Zwecke des Eindrückens einer Brustwarze vorsteht.

2. Stilleinlage nach Anspruch 1, bei der die Erhebung (5) eine Dickstelle des Einlagenkörpers (2) ist.

3. Stilleinlagen nach Anspruch 1 oder 2, bei der Einlagenkörper (2) kuppelförmig gewölbt ist.

4. Stilleinlage nach einem der Ansprüche 1 bis 3, bei der der Einlagenkörper (2) um die Erhebung (5) herum eine konkave innere Oberfläche (3) und/oder bei der der Einlagenkörper (2) eine konvexe äußere Oberfläche (4) aufweist.

5. Stilleinlage nach einem der Ansprüche 1 bis 4, bei der die Wandstärke des Einlagenkörpers (2) vom umlaufenden Außenrand (7) bis zum äußeren Rand der Erhebung (5) allmählich zunimmt.

6. Stilleinlage nach einem der Ansprüche 1 bis 5, bei der die Erhebung (5) zumindest teilweise konvex ist.

7. Stilleinlage nach einem der Ansprüche 1 bis 6, bei der der Übergang von der Erhebung (5) zu dem diese umgebenden Bereich der inneren Oberfläche (3) gerundet ist.

8. Stilleinlage nach einem der Ansprüche 1 bis 7, bei der die Erhebung (5) bezüglich der daran angrenzenden inneren Oberfläche (3) des Einlagenkörpers (2) um 0,5 bis 5mm vorsteht.

9. Stilleinlage nach einem der Ansprüche 1 bis 8, bei der der Einlagenkörper (2) im Bereich der Erhebung (5) eine Wandstärke von 1,5 bis 10mm und/oder außerhalb der Erhebung (5) eine Wandstärke von 0,3 bis 5mm ausweist.

10. Stilleinlage gemäß einem der Ansprüche 1 bis 9, bei der die Erhebung (5) sich vom Zentrum des Einlagenkörpers (2) aus in radialer Richtung 3 bis 12mm weit erstreckt.

11. Stilleinlage nach einem der Ansprüche 1 bis 10, bei der der umlaufende Außenrand (7) des Einlagenkörpers (2) kreisrund oder elliptisch oder blütenförmig ist.

12. Stilleinlage nach einem der Ansprüche 1 bis 11, bei der die Erhebung (5) im Zentrum des Einlagenkörpers (2) angeordnet ist.

13. Stilleinlage nach einem der Ansprüche 1 bis 12, die an der inneren Oberfläche (3) ein Haftmittel aufweist.

14. Stilleinlage nach Anspruch 13, die an der inneren Oberfläche (3) eine Haftschicht (9) aus einem Haftmittel aufweist.

15. Stilleinlage nach einem der Ansprüche 1 bis 14, die anschmiegsam und/oder hautähnlich und/oder atmungsfähig und/oder Flüssigkeit nicht aufnehmend ist.

16. Stilleinlage nach einem der Ansprüche 1 bis 15, bei der der Einlagenkörper (2) aus Silikongummi und/oder das Haftmittel aus einem Silikongel besteht.

## Claims

1. A nursing pad for placement on the breast of a woman with, for the purpose of fitting to the shape of a breast, a thin-walled, flexible pad body (2) with a circumferential outer edge (7), an inner surface (3) for placement on the breast and an outer surface (4), wherein a rising (5) for the purpose of pushing in a nipple protrudes from the inner surface (3) of the pad body (2).

2. The nursing pad according to claim 1, in which the rising (5) is a thickening in the pad body (2).

3. The nursing pad according to claim 1 or 2, in which the pad body (2) is curved in a dome-shaped manner.

4. The nursing pad according to one of claims 1 to 3, in which the pad body (2) around the rising (5) has a concave inner surface (3) and/or in which the pad body (2) has a convex outer surface (4).

5. The nursing pad according to one of claims 1 to 4, in which the wall thickness of the pad body (2) increases gradually from the circumferential outer edge (7) up to the outer edge of the rising (5).

6. The nursing pad according to one of claims 1 to 5, in which the rising (5) is at least partially convex.

7. The nursing pad according to one of claims 1 to 6, in which the transition from the rising (5) to the area of the inner surface (3) surrounding it is rounded.

8. The nursing pad according to one of claims 1 to 7, in which the rising (5) protrudes by 0.5 to 5 mm with respect to the adjacent, inner surface (3) of the pad body (2).

9. The nursing pad according to one of claims 1 to 8, in which the pad body (2) in the area of the rising (5) has a wall thickness of 1.5 to 10 mm and/or outside of the rising (5), a wall thickness of 0.3 to 5 mm.

10. The nursing pad according to one of claims 1 to 9 in which the rising (5) extends 3 to 12 mm from the center of the pad body (2) in the radial direction.

11. The nursing pad according to one of claims 1 to 10, in which the circumferential outer edge (7) of the pad body (2) is circular or elliptical or petal-shaped.

12. The nursing pad according to one of claims 1 to 11, in which the using (5) is arranged in the center of the pad body (2).

13. The nursing pad according to one of claims 1 to 12, which has an adhesive on the inner surface (3).

14. The nursing pad according to claim 13, which has an adhesive layer (9) made of an adhesive on the inner surface (3).

15. The nursing pad according to one of claims 1 to 14, which is supple and/or skin-like and/or breathable and/or does not absorb fluid.

16. The nursing pad according to one of claims 1 to 15, in which the pad body (2) is made of silicone rubber and/or the adhesive is made of a silicone gel.

## Revendications

1. Coussinet d'allaitement destiné à être déposé sur le sein d'une femme avec, à des fins d'adaptation à la forme d'un sein, un corps de coussinet (2) flexible et à paroi de faible épaisseur avec un bord extérieur (7) circonférentiel, une surface intérieure (3) pour le contact avec le sein et une surface extérieure (4), dans lequel une protubérance (5) s'élève, afin de presser un mamelon, de la surface intérieure (3) du corps de coussinet (2).

2. Coussinet d'allaitement selon la revendication 1, dans lequel la protubérance (5) est un endroit épais du corps de coussinet (2).

3. Coussinet d'allaitement selon la revendication 1 ou 2, dans lequel le corps de coussinet (2) est voûté en forme de dôme.

4. Coussinet d'allaitement selon l'une quelconque des revendications 1 à 3, dans lequel le corps de coussinet (2) présente autour de la protubérance (5) une surface intérieure concave (3) et/ou dans lequel le corps de coussinet (2) présente une surface extérieure convexe (4).

5. Coussinet d'allaitement selon l'une quelconque des revendications 1 à 4, dans lequel l'épaisseur de paroi du corps de coussinet (2) s'accroît progressivement du bord extérieur circonférentiel (7) jusqu'au bord extérieur de la protubérance (5).

6. Coussinet d'allaitement selon l'une quelconque des revendications 1 à 5, dans lequel la protubérance (5) est au moins partiellement convexe.

7. Coussinet d'allaitement selon l'une quelconque des revendications 1 à 6, dans lequel la transition de la protubérance (5) à la zone l'entourant de la surface intérieure (3) est arrondie.

8. Coussinet d'allaitement selon l'une quelconque des revendications 1 à 7, dans lequel la protubérance (5) s'élève par rapport à la surface intérieure adjacente (3) du corps de coussinet (2) de 0,5 à 5 mm.

9. Coussinet d'allaitement selon l'une quelconque des revendications 1 à 8, dans lequel le corps de coussinet (2) présente au niveau de la protubérance (5) une épaisseur de paroi de 1,5 à 10 mm et/ou hors de la protubérance (5) une épaisseur de paroi de 0,3 à 5 mm.

10. Coussinet d'allaitement selon l'une quelconque des revendications 1 à 9, dans lequel la protubérance (5) s'étend depuis le centre du corps de coussinet (2) dans le sens radial sur une largeur de 3 à 12 mm.

11. Coussinet d'allaitement selon l'une quelconque des revendications 1 à 10, dans lequel le bord extérieur circonférentiel (7) du corps de coussinet (2) est circulaire ou elliptique ou en forme de pétale.

12. Coussinet d'allaitement selon l'une quelconque des revendications 1 à 11, dans lequel la protubérance (5) est disposée au centre du corps de coussinet (2).

13. Coussinet d'allaitement selon l'une quelconque des revendications 1 à 12, présentant sur la surface intérieure (3) un agent adhésif.

14. Coussinet d'allaitement selon la revendication 13, présentant sur la surface intérieure (3) une couche adhésive (9) consistant en un agent adhésif.

15. Coussinet d'allaitement selon l'une quelconque des revendications 1 à 14, qui est souple et/ou similaire à la peau et/ou respirant et/ou réfractaire aux liquides.

16. Coussinet d'allaitement selon l'une quelconque des revendications 1 à 15, dans lequel le corps de coussinet (2) est en caoutchouc au silicone et/ou l'agent adhésif se compose d'un gel de silicone.
